# EUROPEAN PATENT SPECIFICATION

(11) **EP 3 557 584 B1**
(45) Date of publication and mention of the grant of the patent: **21.08.2024**
(21) Application number: 19167486.0
(22) Date of filing: 05.04.2019
(51) Int. Cl.: G16H 40/67, G16H 15/00, G06F 40/56

(54) **ARTIFICIAL INTELLIGENCE QUERYING FOR RADIOLOGY REPORTS IN MEDICAL IMAGING**
ABFRAGE MIT KÜNSTLICHER INTELLIGENZ FÜR RADIOLOGIEBERICHTE IN DER MEDIZINISCHEN BILDGEBUNG
INTERROGATION D'INTELLIGENCE ARTIFICIELLE POUR RAPPORTS DE RADIOLOGIE EN IMAGERIE MÉDICALE

(30) Priority: 19.04.2018 US 201815957143
(43) Date of publication of application: 23.10.2019
(73) Proprietor: Siemens Healthineers AG, 91301 Forchheim (DE)
(72) Inventor: Sharma, Puneet, Princeton Junction, New Jersey 08550 (US); Xu, Juan, Beijing 100102 (CN)
(74) Representative: Bals & Vogel Patentanwälte PartGmbB

(56) References cited:
- EP-A1- 3 229 157

## Description

### BACKGROUND

The present embodiments relate to radiology reports. Medical reports, such as a radiology report, are primarily a written communication between radiologists, medical professionals, and patients. The reports often contain complex anatomical, technical, and medical information. A comprehensive explanation and deep understanding of the contents of the report has significant value in disease diagnosis, prognosis, and treatment. However, radiology reports can be hard to read, especially for those without a medical background. Patients with limited medical knowledge may benefit from simpler explanation of the report, but such simple explanation may require time and communications with a physician. Medical professionals may not comprehensively understand the report drafted by someone with a different medical expertise or may not desire to again review the entire report to efficiently extract desired information. Document EP 3 229 157 A1 shows a computer-implemented method for predicting answers to questions on medical image analysis reports using a trained recurrent neural network (RNN).

### SUMMARY

By way of introduction, the preferred embodiments described below include methods and systems for obtaining information from a radiology report based on machine learning. Rather than a natural language processing system designed to answer a question based on a large corpus of generic information, deep learning is used to train a machine-learnt network to contribute to extraction of a patient-specific answer from a patient-specific radiology report. Natural language questions, including questions with less informed terminology from a patient and questions with more informed terminology from a physician, are answered using evidence from the patient-specific radiology report even where that report and/or questions are not used in training the machine-learnt network.

The scope of the invention is defined by the appended claims. The present invention is defined in the independent claims. The embodiments are defined in the dependent claims.

### BRIEF DESCRIPTION OF THE DRAWINGS

The components and the figures are not necessarily to scale, emphasis instead being placed upon illustrating the principles of the invention. Moreover, in the figures, like reference numerals designate corresponding parts throughout the different views.
Figure 1 is a flow chart diagram of an alternative embodiment of a method for obtaining information from a radiology report based on machine learning;
Figure 2 is a flow chart diagram of the method according to the invention for obtaining information from a radiology report based on machine learning;
Figure 3 illustrates an example template to be populated with parsing from a radiology report;
Figure 4 illustrates an example question, results from question analysis, answer, and evidence;
Figure 5 is a flow chart diagram of yet another embodiment of a method for obtaining information from a radiology report based on machine learning;
Figure 6 illustrates an example table showing evidence extraction from candidate sentences;
Figure 7 is a block diagram of the system according to the invention for obtaining information from a radiology report based on machine learning; and
Figure 8 illustrates an example question and candidate sentences identified as having a top probability of evidence.

### DETAILED DESCRIPTION OF THE DRAWINGS AND PRESENTLY PREFERRED EMBODIMENTS

Question answering (QA) systems generally use a combination of techniques from computational linguistics, information retrieval and knowledge representation for finding answers. QA systems have been studied and showed success in various research areas. For example, IBM Watson QA system defeated two Jeopardy champions. EAGLi QA system is a search engine for MEDLINE with terminology-powered navigation and knowledge extraction skills. Facebook research DrQA system is a reading comprehension system for Wikipedia to answer open-domain questions. START is a web-based QA system to answer various questions about geography, arts, history, and other generic subjects. Most existing QA systems are at a research level and use a large database of generic information.

For a patient's radiology report, a QA system answers questions specific to that single source. Artificial intelligence-based querying of knowledge from radiology reports may assist patients and medical professionals in understanding or efficiently using a radiology report for a specific patient. The patient-specific QA system is based on natural language processing (NLP), deep learning techniques. Given a natural language question together with a document (i.e., radiology report), the QA system can understand the question and document, automatically retrieve the corresponding information as the evidence, and generate the answer.

Question-answering-based interaction automatically answers questions posed by humans in natural language. Various audiences are supplied with just right information specific to a radiology report of a given patient. Clinical language is not easy to understand for most patients. The QA system provides an easy way to convey the radiology report to patient. The patient may use their vocabulary to ask any questions, which the QA system answers based on the patient's radiology report. The radiology report QA system may pull answers from an unstructured document (i.e., free-form report). The QA system is a flexible analysis tool for users with various purposes, such as medical professionals, financial staffs, insurance companies and/or patients.

For analysis over multiple patients (e.g., physician and/or hospital performance study), the same question or questions are asked for many radiology reports. Key information is automatically retrieved from the large volume of data. The key information may be used to generate labels at various anatomical sites for corresponding images for the radiology reports. Comparing with manual annotation, this auto-annotation mechanism may improve the efficiency in development of big image data analysis. The use of machine-learnt networks allow the computer to more quickly provide an answer over a broader range of questions and vocabulary. The arrangement of such networks and sequence allow a machine to more accurately provide an answer specific to a patient's radiology report.

The QA system is not limited to radiology reports. Other patient-specific text data, such as medical record, family history, lab test report, and so on, may be used for answering a question.

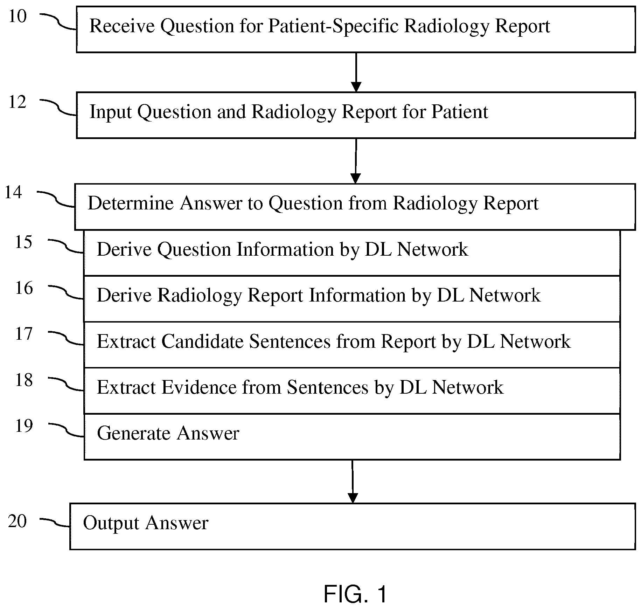

Figure 1 shows an alternative embodiment of a method for obtaining information from a radiology report based on machine learning. Deep machine learning trains one or more machine-learnt networks. The machine-learnt networks are trained to contribute to natural language processing in a QA system, such as for analyzing the question, parsing the radiology report, matching text from the radiology report to the question, extracting evidence from the text, and/or determining an answer from the extracted evidence. The QA system is configured, in part by the deep learning, to answer a question specific to the content of one document, the radiology report.

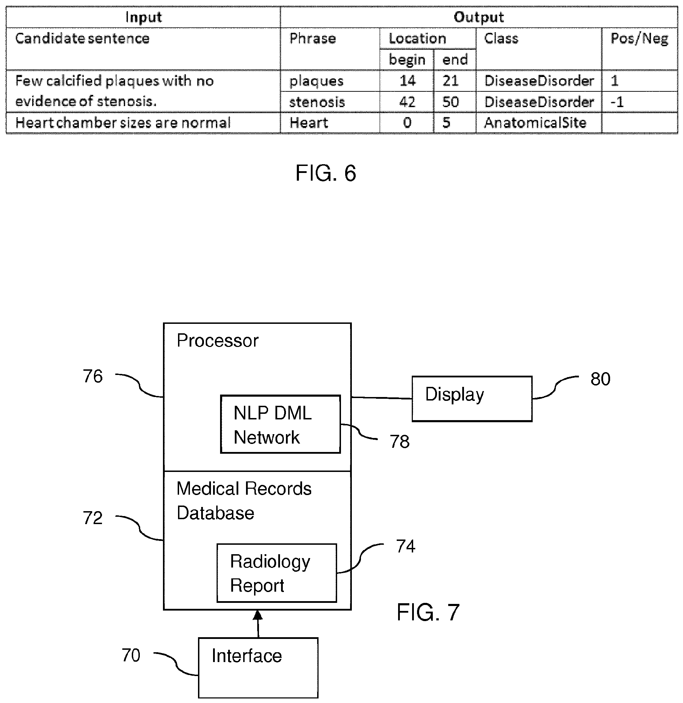

The method not part of the invention is implemented by the system of Figure 7 or another system. For example, the method is implemented by a computer, server, or other processor. A radiology report is loaded from memory. An interface receives the question. A computer (e.g., processor) applies the machine-learnt network and any other natural language processing (QA system) based on the question and the radiology report. The computer outputs an answer to the question based on the information in the input radiology report. The output answer may be stored in memory or displayed on a display device. Different devices may be used.

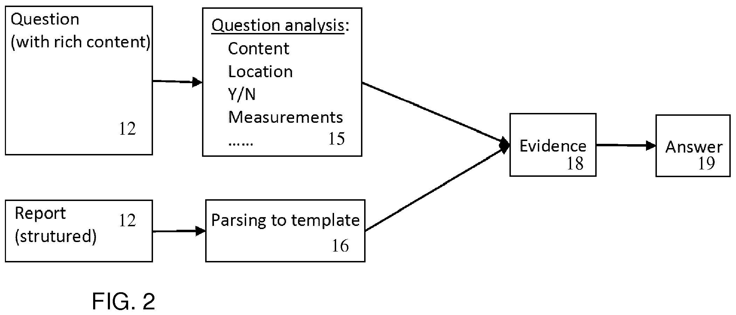

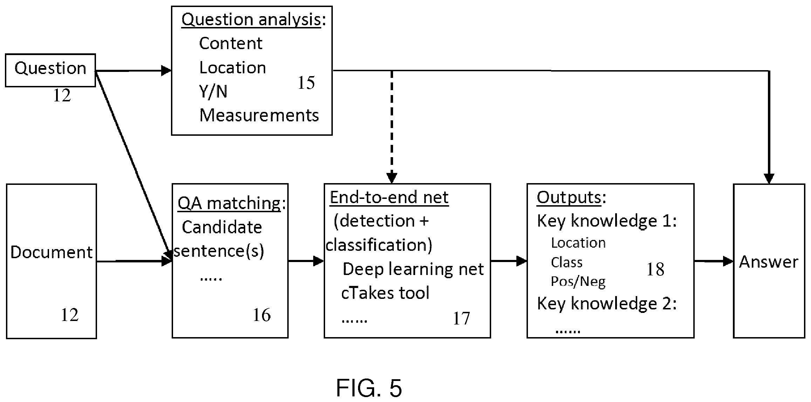

Additional, different, or fewer acts may be provided. For example, the question is generated rather than received in act 10, such as where a computer generates a series of questions for analysis. In another example, acts 15-19 represent one example for determining an answer to a question about a given radiology report. Less than all and/or additional acts may be used to determine the answer. Figures 2 and 5 shows two other embodiments, where the method of Figure 2 does not extract sentences in act 17 and the method of Figure 5 does not parse to a template.

Acts 15-18 are shown as using deep-learnt networks. Different deep learnt networks or classifiers are used for each act, but a common network or a network formed from other networks may be used for implementing two or more acts. In other embodiments, a deep-learnt network is not used for one or more of acts 15-18 and/or is used for act 19.

The acts are performed in the order shown (e.g., top to bottom or numerical) or other orders. For example, acts 15 and 16 are performed in a reverse order or simultaneously.

In act 10, interface hardware receives a question about a patient. The interface hardware is a communications interface, such as a user input device, port, bus, or memory. The question is received by the interface hardware, such as receiving a question typed on a keyboard at a universal serial bus (USB) connector of a computer. A microphone may be used to receive the question.

The question is input as text or converted to text. The question is a natural language question, such as using phrasing, abbreviations, terms, and/or structure in a common language, such as English. The question is not a pre-prepared question, such as the user selecting a question from a list (e.g., from a table or drop-down menu). In alternative embodiments, the question is selected from a list.

The question is patient-specific. Rather than being generic to many patients, the question is about a particular patient. The question is directed to or for a given patient. The same question may be asked for other patient. For example, the question is whether there is an indication of stenosis of a right coronary artery for the patient. In another example, the question is "what is the level of calcification of plaque?" In other embodiments, the question is not about a specific patient, but instead is a question that is asked for many patients.

The question is received from the patient. The phrasing of the question may be different due to being from a patient, such as whether there is an indication of a heart problem rather than whether there is an indication of stenosis of a right coronary artery. Any vocabulary and phrasing may be used. Rather than having to contact a physician, the patient or a relative may ask a question, and the QA system provides an answer based on the patient's radiology report.

In the embodiment according to the invention, the question is received from a treating physician. A surgeon, anesthesiologist, primary care physician (internal medicine), nurse, or other medical professional asks the question. The phrasing may be appropriate for the physician or medical professional. Since the medical professional may have different knowledge than the radiologist that prepared the report and/or to avoid having to review the entire report again to find an answer, the QA system answers the question from the medical professional based on the report for the patient being treated.

In another embodiment, the questions are posed by an algorithm. A computer generates one or more questions. Computer-generated questions may be used for machine training or for analysis of answers. For example, data analytics are performed for radiology reports for a physician and/or hospital. Key or embedded knowledge is automatically retrieved in the large volume of radiology reports. The question is loaded from memory or otherwise generated, and the answers are used for radiology research and/or to improve practices in reporting.

In act 12, the interface, memory, and/or processor inputs the question and a radiology report into the QA system. The patient-specific question and the radiology report for that patient are input to the natural language processing system for an answer.

The natural language processing system includes a deep machine-learnt network. Other natural language processing tools may also be included. Computational linguistics (e.g., linguist rules), information retrieval, and/or knowledge representation may be part of the natural language processing system.

The deep machine-learnt network contributes to any part of the natural language processing system. The network contributes to extraction of an answer from the patient-specific input (e.g., the radiology report for the patient and/or question about the patient). Any contribution may be provided, such as for analyzing the question, parsing the report, extracting candidate phrases or sentences from the report, retrieving evidence from the report and/or extracted information, and/or generating an answer from retrieved evidence. One network may be trained to perform all these acts. A separate network may be trained for each of the acts. Some acts may not use any machine-learnt network.

The deep machine-learnt network is any now known or later developed network. In general, deep learning uses a neural network with raw data or data without conversion to other features as input and a ground truth. The relationship of the values of the input raw data to the ground truth is learned. For example, deep learning (e.g., deep structured learning, hierarchical learning, or deep machine learning) models high-level abstractions in data by using multiple processing layers with structures composed of multiple non-linear transformations, where the input data features are not engineered explicitly. Deep learning may be used without including specific linguistic rules. A deep neural network processes the input via multiple layers of feature extraction to produce features used to output. The deep learning provides the features used to generate the output. In alternative embodiments, other machine learning is used, such as a Bayesian network or support vector machine.

For natural language processing, the deep-machine learnt network may be a recursive neural network and/or a long term-short memory network. Any architecture may be used. Other deep learnt, sparse auto-encoding models may be trained and applied. The machine training is unsupervised in learning the features to use and how to classify given an input sample (i.e., feature vector). The combination of information in the raw data indicative of the ground truth is learned, and an output given input combination of information is learned.

For training, the training data includes many samples. The samples are based on the contribution. The samples include appropriate input information and corresponding ground truth or output to be learned. For example, many different questions in the form of ASCI text are provided as samples of the raw data to be input. The ground truth is the question information, such as content (e.g., category of subject matter, object, or subject), type of question (Yes/No, how, what, where ...), anatomy, and/or anatomy location. The deep learning learns to provide the question information from an input question. In another example, many samples of radiology reports are used with the content of the reports in a structural template as the ground truth. The deep learning learns to provide the structural template populated with the content of the reports. As another example, many samples of question information and report sentences are provided with particular ones of the report sentences having content relative to the question information as ground truth. As yet another example, many samples of question information are provided as raw data inputs and corresponding text from medical reports or templates matching the question information are provided as the ground truth. The deep learning learns to provide key knowledge (e.g., evidence) from the text. In yet another example, many samples of extracted evidence and corresponding question information are provided with a correct answer as the ground truth. As a final example, many samples of questions and radiology reports are provided with the correct answer as the ground truth. The network is trained to provide the answer from the question and report without intervening processing. Other inputs and ground truths (i.e., outputs) may be used.

The trained network is stored in a memory. The trained artificial intelligence (i.e., machine-learnt network) is stored. The result of the training is a matrix or other model. The matrix represents the learned knowledge through machine training using deep learning. Other machine-learnt network representations may be used, such as a hierarchy of matrices or other non-linear models.

Any memory may be used. The memory used for the training data may be used. For application, the memory may be in other devices. For example, the trained model is stored in a memory of a server. The server uses the trained model to output to clients. As another example, multiple copies of the trained model are provided to different physicians, medical scanners, and/or workstations for use by different physicians.

Once trained, the machine-learnt network is applied by a machine, such as a computer, processor, or server. The machine uses input raw data for a patient (i.e., radiology report, question, and/or information derived therefrom) and the machine-learnt network to generate an output, such as question information, a parse of the report, evidence, and/or an answer.

The only inputs to the QA system are the question and the radiology report for the patient. Rather than answering the question based on a corpus that includes reports for other patients, only the radiology report or reports for the specific patient are input. The QA system may include learned knowledge from the deep learning, but that knowledge is not input during application to answer a question. The deep learnt knowledge is applied to answer the question. In an alternative embodiment, other information is input to answer the question, such as other information from a medical record of the patient and/or information generic to patients (e.g., a medical atlas).

For application for a specific patient, the input question is different than any question used to train the deep machine-learnt network. At least one word or order of words in the question is different. Similarly, the input radiology report for the patient is different than any radiology report used to train the deep machine-learnt network. At least the text, image, or organization is different. By deep learning by the machine, the machine-learnt network is trained to predict an output based on the input even if the input is different than any used in training. In alternative embodiments, the question and/or radiology report input during application is the same as used for training. Rather than looking up the output in a database, the machine-learnt network outputs an answer based on the training.

In act 14, the computer (e.g., processor) determines an answer to the input question. In response to the input of the question and the radiology report, an answer to the question is derived from the radiology report. Only the information from the radiology report for the specific patient is used to derive the answer to the question. A patient-specific answer is determined as specific to the radiology report for that patient. Alternatively, other information is also used, such as atlas information.

The answer is determined by the QA system with natural language processing including the deep machine-learnt network. The deep machine-learnt network is part of the natural language processing, such as to retrieve evidence in response to a natural language question about a patient from a radiology report for the patient and/or forming an answer from the evidence. The deep machine-learnt network is a natural language processing deep-learnt network. Other natural language processing may be applied, such as linguistic rules, information retrieval, knowledge representation, text/sentence negation, sentiment analysis, and/or logical reasoning.

The natural language processing, in general, performs multiple acts to derive the answer to the question from the radiology report. Question information is derived from the question using natural language processing. The radiology report is reformatted, parsed, used to populate a template, and/or separated using natural language processing. Evidence and/or candidate sentences or phrases relevant to the question are identified using natural language processing. The evidence and/or candidates are analyzed using natural language processing to provide an answer.

Additional, different, or fewer acts may be provided. Acts 15-19 show one example combination of acts using natural language processing. Figures 2 and 5 show other example embodiments of combinations of acts to derive an answer using natural language processing.

The deep machine-learnt network or networks are used in one or more of the acts. Machine-learnt networks may be used in all or only a sub-set of the acts.

In act 15, question information is derived from the question. Any question information may be derived. The content (e.g., subject, object, and/or category of subject matter), type of question (e.g., Yes/no, compound, who, what, where, how, why, when, ...), anatomy, anatomy location, or other question information is extracted from the question. For example, the question is input as "is there a stenosis in the RCA?" The content is identified as coronary arteries; the type of question is Yes/No; a sign/measure/symptom is stenosis; and the anatomy/organ/segment is right coronary artery. Other medical related categorization and/or content may be derived, such as association with a branch or group of terms.

Natural language processing is used to extract the question information. Some or all the question information may be derived by a deep machine-learnt network. The question is analyzed to determine the question information.

In acts 16, radiology report information is derived from the radiology report. The radiology report is analyzed to understand the document. Sections or types of information may be identified. The content, anatomy, and/or other information about each part may be derived. Different parts of the radiology report are separated, such as identifying sentences, clauses or phrases. In one embodiment, the radiology report is split into sentences, but other separations (e.g., by section or grammar-based) may be used.

In the embodiment according to the invention, the radiology report is parsed. The parsing locates different types of information and places the information into a template radiology report. The template includes a hierarchical standardized structure, which is populated from the radiology report based on natural language processing. In another embodiment, grammatical rules or searching for punctuation and/or capitalization is used to extract the parts of the radiology report. Other derivations may be performed.

Natural language processing is used to derive the radiology report information. For example, computational linguistics or knowledge representation are used to extract. Some or all the radiology report information may be derived by a deep machine-learnt network.

In act 17, candidate sentences or phrases are extracted from the radiology report and/or from the radiology report information from act 16. The information or parts of the radiology report corresponding to the question information are extracted. For example, the sentences are compared to the question, and the candidate sentences that relate to the question are extracted (e.g., any sentence with content for right coronary artery is identified). Less than all the sentences are extracted as candidate sentences. As another example, the parts of the hierarchal template structure corresponding to the question are extracted.

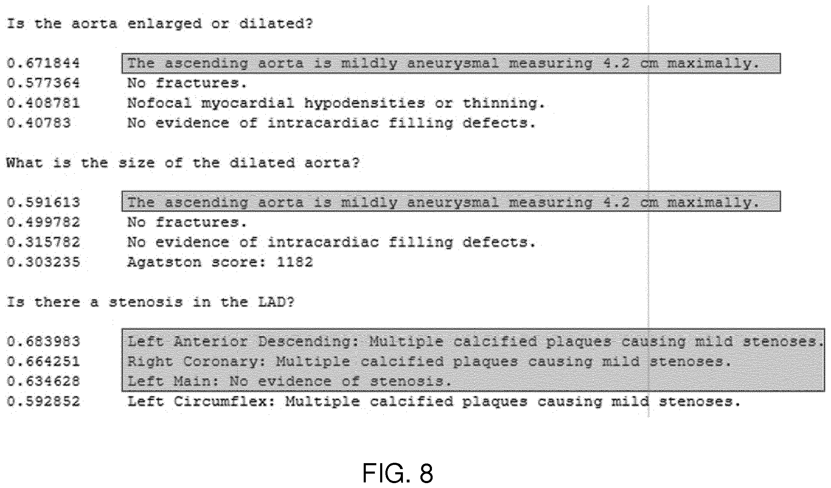

Natural language processing is used to extract from the radiology report information associated with or providing evidence for the question. A deep machine-learnt network may be applied to extract. The radiology report information that relates to the question information is output as a match. Figure 8 shows an example, where three different questions and the corresponding four candidate sentences having the top four probabilities (shown) are listed.

In act 18, evidence is extracted from the radiology report information and/or extracted candidates therefrom. Any evidence that may contribute to the answer is extracted. The candidate sentences and/or radiology report information may be parsed or analyzed with respect to the question information. For example, a candidate sentence is "Right Coronary: No evidence of stenosis." The extracted evidence is a negative with respect to stenosis and the right coronary artery.

Natural language processing is used to extract the evidence. A deep machine-learnt network may be applied to extract the evidence. The evidence in the radiology report information and/or candidates that indicate a possible answer to the question is retrieved by the natural language processing.

In act 19, an answer is generated from the evidence, candidates, and/or radiology report information. The evidence retrieved from the radiology report is analyzed with respect to the question information to generate the answer. For example, the evidence is combined to determine the answer.

Natural language processing is used to generate the answer. The evidence is combined, such as probabilistically, to determine a most likely or probable answer. Rules or fuzzy logic may be used. A deep-machine learnt network may be used to generate the answer, such as outputting possible answers and corresponding probabilities given input evidence and question information. The possible answer with a greatest probability is selected. Alternatively, the network may output a single answer with or without probability information.

In act 20, the computer (e.g., processor) outputs the answer. The answer provided by the determination is output. The output is to a display device, such as a response to input of the question on a user interface. Alternatively, the output is to a report, such as adding the question and answer to the radiology report and/or the medical data record of the patient.

The use of a deep machine-learnt network may make the response time of the computer to provide an answer more rapid. Due to the application of the network rather than hand programmed series of calculations, the computer operates more quickly to provide an answer.

The output answer may assist a patient or family in understanding their radiology report. Rather than having to search for meanings of medical terms, simple language questions may be posed and simple language answers output. Arranging for consultation with a physician may be avoided.

The output answer may assist a physician. Reviewing the entire radiology report again may be avoided by asking a question and receiving the answer. Where the radiologist uses different expressions or terminology, the physician may receive an answer that avoids confusion, assisting in diagnosis, prognosis, and/or treatment.

Figures 2-4 show an example embodiment of the method of Figure 1. A template matching approach is used, so the arrangement allows the QA system to operate based on structured language. The input question has sufficient information to form an answer, such as including radiology related anatomy. The input report uses structured language, so may be parsed or grammatically analyzed.

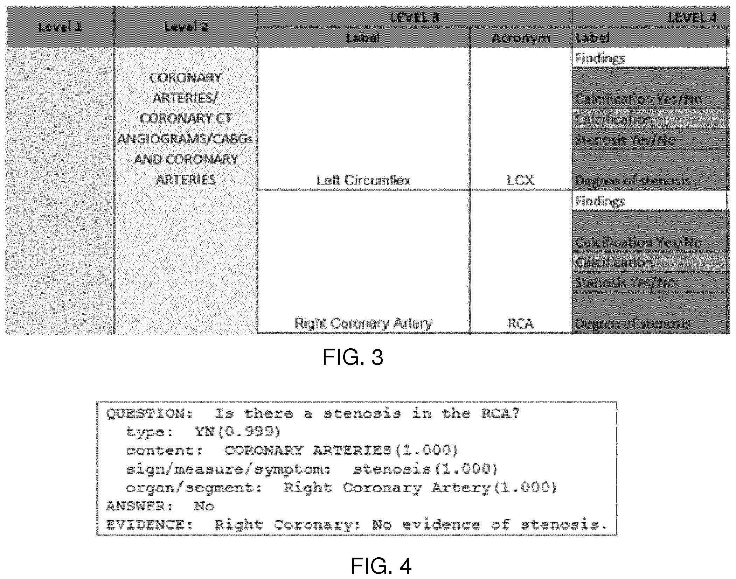

A question with rich content and a radiology report are input in act 12. In act 15, question information (content, type, and anatomy location) are extracted from the question by a deep machine-learnt classifier. The input question is analyzed to understand the content, type, location etc. In act 16, the radiology report is parsed into a template by another deep machine-learnt classifier. The template has a hierarchical standardized structure. Figure 3 shows an example part of the template, where different levels correspond to the hierarchy, ending with a level with findings. In act 18, text from the template is selected based on the question information. The question information (e.g., content, type, and/or anatomy location) is matched into the template to select the corresponding text as evidence. In act 19, the answer is generated from the selected text. The question is matched into the template to pick-up the right answer. The selected text correlates to findings, which are used to answer the question.

Figure 4 shows the outputs of the acts of Figure 2 in one example. The evidence is the selected text. The numbers by the question information represent probabilities associated with the information. Where the probability is 1.0, there is no contrary indication.

Figures 5 and 6 show another example embodiment of the method of Figure 1. Template matching is not used, so there are no requirements on the input document. This allows the computer to operate the QA system with text, graphics, charts, medical images, and/or spreadsheets. Structure and/or unstructured data may be used. The input question may be more flexible in content, and the QA system may better handle natural language than the embodiment of Figure 2.

A question and a radiology report are input in act 12. In act 15, question information (content, type, anatomy location, and measurements) are extracted from the question by a natural language processing deep machine-learnt classifier. The input question is analyzed to understand the content, type, location etc. In act 16, the radiology report is separated into sentences or phrases. Grammatical rules, punctuation rules, or other hand programmed rules are used to split the radiology report into sentences or phrases. In act 17, candidates of the sentences or phrases are identified by another natural language processing deep machine-learnt classifier. The question and report sentences are fed into a QA matching model (e.g., a pre-trained deep learning model). The candidate sentences containing answers based on the question information are automatically detected by the model as the evidence. Natural language processing tools, such as cTakes, may be used to extract the evidence. In act 18, parts of the candidates are selected. The candidate sentences are analyzed using natural language processing tools and/or a deep machine-learnt network to extract evidence (e.g., key knowledge) from the candidate sentences. Specific information from the statements that relates to the question is identified. In act 19, the answer is generated from the selected text. The question is matched into the evidence to pick-up the right answer. The selected evidence correlates to an answer to the question.

Figure 6 shows the outputs of some of the acts of Figure 5 in one example (e.g., extraction of key knowledge from the candidate sentences). Two candidate sentences are shown. Text or phrase from the candidate sentences extracted as evidence are shown. The location is a text location in the sentence represented numerically. The classification and positive/negative are further extracted evidence at those locations.

Figure 7 shows a block diagram of the system according to the invention for obtaining information from a radiology report based on machine learning. The system is for application of the machine-learnt model but may alternatively or additionally be used for training with machine learning. Using deep learning, question information, template population, report parsing, matching of the question to candidates, text, or other evidence, and/or matching the evidence to an answer may be performed more rapidly by the system. The system implements a QA and/or natural language processing system able to derive answers to questions based on patient-specific radiology reports.

The system implements the method of Figure 1, the method of Figure 2, the method of Figure 5, and/or other natural language processing using a deep machine-learnt network to contributed to answering a question for a specific patient. Other methods or acts may be implemented, such as acts for selecting the radiology report and/or using the answer.

The system includes an interface 70, a medical records database 72 with a radiology report 74, a processor 76 for applying a natural language processing, deep machine-learnt network 78, and a display 80. Additional, different, or fewer components may be provided. For example, a medical imager is provided for scanning the patient to generate the radiology report 74. In another example, a network or network connection is provided, such as for networking with a medical imaging network or data archival system or interconnecting the processor 76 and the database 72.

The interface 70, medical records database 72, processor 76, and/or display 80 are part of a medical imager, server, workstation, or computer. In one embodiment, the interface 70, medical records database 72, processor 76, and/or display 80 are a personal computer, such as desktop or laptop. In yet other embodiments, the medical records database 72 is part of a separate computer from the processor 76.

The interface 70 is a communications port, such as an ethernet card. In other embodiments, the interface 70 is a user interface, such as user input device (e.g., keyboard, mouse, trackpad, touchscreen, and/or roller ball). The interface 70 may be a bus, chip, or other hardware for receiving the radiology report and/or question.

The interface 70 is configured to receive a question about a radiology report of a patient. The configuration is provided by software, hardware, and/or firmware. For example, the interface 70 is configured by an operating system to receive user entry of a question via audio or text.

The medical records database 72 is a random access memory, system memory, cache memory, hard drive, optical media, magnetic media, flash drive, buffer, database, combinations thereof, or other now known or later developed memory device for storing the radiology report 74, the deep machine-learnt network 78, and/or data generated by natural language processing (e.g., template, sentences, candidates, evidence, text, probabilities, and/or answer). The medical records database 72 is part of the computer associated with the processor 76 or is a separate or remote database for access over a computer network.

The medical records database 72 or other memory is alternatively or additionally a non-transitory computer readable storage medium storing data representing instructions executable by the programmed processor 76 for learning or applying the machine-learnt model, and/or for natural language processing. The instructions for implementing the processes, methods, and/or techniques discussed herein are provided on non-transitory computer-readable storage media or memories, such as a cache, buffer, RAM, removable media, hard drive, or other computer readable storage media. Non-transitory computer readable storage media include various types of volatile and nonvolatile storage media. The functions, acts or tasks illustrated in the figures or described herein are executed in response to one or more sets of instructions stored in or on computer readable storage media. The functions, acts or tasks are independent of the particular type of instructions set, storage media, processor or processing strategy and may be performed by software, hardware, integrated circuits, firmware, micro code and the like, operating alone, or in combination. Likewise, processing strategies may include multiprocessing, multitasking, parallel processing, and the like.

In one embodiment, the instructions are stored on a removable media device for reading by local or remote systems. In other embodiments, the instructions are stored in a remote location for transfer through a computer network or over telephone lines. In yet other embodiments, the instructions are stored within a given computer, CPU, GPU, or system.

The processor 76 is a general processor, control processor, digital signal processor, application specific integrated circuit, field programmable gate array, or other hardware processor for natural language processing in a QA system. The processor 76 is part of a computer, workstation, server, or other device configured to apply machine learning and/or to apply a machine-learnt network 78. The processor 76 is configured by software, hardware, and/or firmware. For learning, the processor 76 is configured by one or more machine learning algorithms. For applying a learnt network 78, the processor 76 is configured, in part, by a learnt matrix or matrices, table, or other neural network representation associating input data to output data.

The processor 76 applies the machine-learnt network 78 and/or other natural language processing. The machine-learnt network 78, as implemented by the processor 76, contributes to determining an answer to a question from a radiology report. The processor 76 is configured to analyze the question for information, identify parts of the radiology report, retrieve evidence related to the question from the parts, and generate an answer to the question based on the evidence. The configuration for at least one of the analyze, identify, retrieve, or generate includes natural language processing and/or application of a natural language processing, deep training-based machine-learnt network.

In the embodiment according to the invention, the processor 76 is configured to: analyze by extraction of the information as content, type, and anatomy location by a first deep machine-learnt classifier of the deep-training based machine-learnt network; identify the parts by a parse of the radiology report into a template by a second deep machine-learnt classifier of the deep-training based machine-learnt network; retrieve the evidence as a selection of text from the template based on the content, type, and/or anatomy location; and/or generate the answer from the selected text.

In another embodiment, the processor 76 is configured to: extract content, type, and anatomy location from the question with a first deep machine-learnt classifier of the deep-training based machine-learnt network; separate the radiology report into sentences or phrases; identify candidates of the sentences or phrases by a second deep machine-learnt classifier of the deep-training based machine-learnt network; select parts of the sentences or phrases; and/or generate the answer from the selected parts.

The display 80 is a monitor, LCD, projector, plasma display, CRT, printer, or other now known or later developed device for displaying the answer, question, evidence, and/or radiology report. The display 80 receives the answer from the processor 76, the radiology report from the processor 76 or database 72, and the question from the interface 70 or processor 76. The processor 76 formats the data into an image and stores the image in a buffer, configuring the display 80. The display 80 uses the image in the buffer to generate an image for viewing. The image includes graphics, alphanumeric text, anatomical scan, and/or other information for communicating the answer.

## Claims

1. A computer-implemented method for obtaining information from a radiology report (74) based on machine learning, the method comprising:
receiving (10) from interface (70) hardware a question about a patient, wherein receiving (10) comprises receiving (10) the question from the patient or wherein receiving (10) comprises receiving (10) the question from a treating physician of the patient and wherein the interface (70) hardware is a user interface;
inputting (12) the question and a radiology report (74) from medical imaging of the patient into a natural language processing system, the natural language processing system comprising a deep machine-learnt network having been trained to contribute to extraction of an answer from patient-specific input;
determining (14) an answer to the question in response to the inputting (12) and using the deep machine-learnt network, the answer specific to the radiology report (74) for the patient; and
outputting (20) the answer, wherein the output is to a display device, such as a response to the input of the question on the user interface, wherein the deep machine-learnt network comprises at least first and second deep machine-learnt classifiers, and wherein determining (14) comprises:
extracting (15) content, type of the question, anatomy location and measurements from the question by the first deep machine-learnt classifier and analyzing the question to understand the content, the type of the question, the anatomy location, and the measurements,
parsing (16) the radiology report (74) into a template by the second-deep machine-learnt classifier, wherein the template includes a hierarchical standardized structure comprising different levels, ending with a level with findings, and wherein the template is populated from the radiology report based on the natural language processing,
selecting (18) text from the template based on the content, the type of the question, the anatomy location, and the measurements, wherein the content, the type of the question, the anatomy location and the measurements are matched into the template to select the corresponding text as evidence, and
generating (19) the answer from the selected text, wherein the question is matched into the template to pick-up the right answer, wherein the selected text correlates to findings, which are used to answer the question.

2. The method according to claim 1, wherein determining (14) comprises deriving (15) question information from the question by the deep machine-learnt network
and/or
wherein determining (14) comprises deriving (16) radiology report (74) information from the radiology report (74) by the deep machine-learnt network.

3. The method according to any of the preceding claims, wherein determining (14) comprises extracting (17) sentences or phrases from the radiology report (74) by the deep machine-learnt network
and/or
wherein determining (14) comprises extracting (18) evidence from sentences or phrases from the radiology report (74), the evidence extracted by the deep machine-learnt network.

4. The method according to any of the preceding claims, wherein inputting (12) comprises inputting (12) only the question and the radiology report (74) and wherein determining (14) comprises determining (14) the answer only from information in the radiology report (74),
or
wherein inputting (12) the question comprises inputting (12) the question where the question is different than any question used to train the deep machine-learnt network, and wherein inputting (12) the radiology report (74) comprises inputting (12) the radiology report (74) where the radiology report (74) is different than any radiology report (74) used to train the deep machine-learnt network,
or
wherein inputting (12) the question comprises inputting (12) as text without selection from a list.

5. A system for obtaining information from a radiology report (74) based on machine learning, the system comprising:
an interface (70) configured to receive a question about a radiology report (74) of a patient, wherein receiving (10) comprises receiving (10) the question from the patient or wherein receiving (10) comprises receiving (10) the question from a treating physician of the patient and wherein the interface (70) is a user interface;
a medical records database (72) having stored therein the radiology report (74) of the patient;
a processor (76) configured to:
inputting (12) the question and a radiology report (74) from medical imaging of the patient into a natural language processing system, the natural language processing system comprising a deep machine-learnt network having been trained to contribute to extraction of an answer from patient-specific input;
determining (14) an answer to the question in response to the inputting (12) and using the deep machine-learnt network, the answer specific to the radiology report (74) for the patient; and
wherein the deep machine-learnt network comprises at least first and second deep machine-learnt classifiers, and wherein determining (14) comprises:
extracting (15) content, type of the question, anatomy location and measurements from the question by the first deep machine-learnt classifier and analyzing the question to understand the content, the type of the question, the anatomy location, and the measurements,
parsing (16) the radiology report (74) into a template by the second-deep machine-learnt classifier, wherein the template includes a hierarchical standardized structure comprising different levels, ending with a level with findings, and wherein the template is populated from the radiology report based on the natural language processing,
selecting (18) text from the template based on the content, the type of the question, the anatomy location, and the measurements, wherein the content, the type of the question, the anatomy location and the measurements are matched into the template to select the corresponding text as evidence, and generating (19) the answer from the selected text, wherein the question is matched into the template to pick-up the right answer, wherein the selected text correlates to findings, which are used to answer the question; and
a display (80) configured to output the answer, such as a response to the input of the question on the user interface.

6. The system according to claim 5, wherein the deep training-based machine learnt network (78) comprises a long term short memory network.

## Patentansprüche

1. Computerimplementiertes Verfahren zur Gewinnung von Informationen aus einem Radiologiebericht (74) auf der Grundlage von maschinellem Lernen, wobei das Verfahren umfasst:
Empfangen (10) einer Frage über einen Patienten von der Hardware einer Schnittstelle (70), wobei das Empfangen (10) das Empfangen (10) der Frage von dem Patienten umfasst oder wobei das Empfangen (10) das Empfangen (10) der Frage von einem behandelnden Arzt des Patienten umfasst und wobei die Hardware der Schnittstelle (70) eine Benutzerschnittstelle ist;
Eingeben (12) der Frage und eines Radiologieberichts (74) aus der medizinischen Bildgebung des Patienten in ein System zur Verarbeitung natürlicher Sprache, wobei das System zur Verarbeitung natürlicher Sprache ein tiefes maschinelles Netzwerk umfasst, das so trainiert wurde, dass es zur Extraktion einer Antwort aus einer patientenspezifischen Eingabe beiträgt;
Bestimmen (14) einer Antwort auf die Frage als Reaktion auf das Eingeben (12) und unter Verwendung des tiefen maschinell erlernten Netzwerks, wobei die Antwort spezifisch für den Radiologiebericht (74) für den Patienten ist; und
Ausgeben (20) der Antwort, wobei die Ausgabe an eine Anzeigevorrichtung erfolgt, beispielsweise als Antwort auf die Eingabe der Frage auf der Benutzerschnittstelle,
wobei das tiefe maschinell erlernte Netzwerk mindestens einen ersten und einen zweiten tiefen maschinell erlernten Klassifikator umfasst, und wobei das Bestimmen (14) umfasst:
Extrahieren (15) des Inhalts, der Art der Frage, der Lage der Anatomie und der Maße aus der Frage durch den ersten tiefen maschinell erlernten Klassifikator und Analysieren der Frage, um den Inhalt, die Art der Frage, die Lage der Anatomie und die Maße zu verstehen,
Kopieren (16) des Radiologieberichts (74) in eine Schablone durch den zweiten tiefen maschinell erlernten Klassifikator, wobei die Schablone eine hierarchische standardisierte Struktur mit verschiedenen Ebenen umfasst, die mit einer Ebene mit Befunden endet, und wobei die Schablone aus dem Radiologiebericht auf der Grundlage der natürlichen Sprachverarbeitung ausgefüllt wird,
Auswählen (18) von Text aus der Schablone auf der Grundlage des Inhalts, der Art der Frage, der anatomischen Position und der Messungen, wobei der Inhalt, die Art der Frage, die anatomische Position und die Messungen in die Schablone eingepasst werden, um den entsprechenden Text als Beweis auszuwählen, und
Generieren (19) der Antwort aus dem ausgewählten Text, wobei die Frage mit der Vorlage abgeglichen wird, um die richtige Antwort zu finden, wobei der ausgewählte Text mit Erkenntnissen korreliert, die zur Beantwortung der Frage verwendet werden.

2. Verfahren nach Anspruch 1, wobei das Bestimmen (14) das Ableiten (15) von Frageinformationen aus der Frage durch das tiefe maschinell erlernte Netzwerk umfasst
und/oder
wobei das Bestimmen (14) das Ableiten (16) von Informationen des Radiologieberichts (74) aus dem Radiologiebericht (74) durch das tiefe maschinell erlernte Netzwerk umfasst.

3. Verfahren nach einem der vorhergehenden Ansprüche, wobei das Bestimmen (14) das Extrahieren (17) von Sätzen oder Phrasen aus dem Radiologiebericht (74) durch das tiefe maschinell erlernte Netzwerk umfasst
und/oder
wobei das Bestimmen (14) das Extrahieren (18) von Beweisen aus Sätzen oder Phrasen aus dem Radiologiebericht (74) umfasst, wobei die Beweise durch das tiefe maschinell erlernte Netzwerk extrahiert werdenk.

4. Verfahren nach einem der vorhergehenden Ansprüche, wobei das Eingeben (12) nur das Eingeben (12) der Frage und des Radiologieberichts (74) umfasst und wobei das Bestimmen (14) das Bestimmen (14) der Antwort nur aus der Information in dem Radiologiebericht (74) umfasst,
oder
wobei das Eingeben (12) der Frage das Eingeben (12) der Frage umfasst, wobei sich die Frage von jeder Frage unterscheidet, die zum Trainieren des tiefen maschinell erlernten Netzwerks verwendet wird, und wobei das Eingeben (12) des Radiologieberichts (74) das Eingeben (12) des Radiologieberichts (74) umfasst, wobei sich der Radiologiebericht (74) von jedem Radiologiebericht (74) unterscheidet, der zum Trainieren des tiefen maschinell erlernten Netzwerks verwendet wird,
oder
wobei das Eingeben (12) der Frage das Eingeben (12) als Text ohne Auswahl aus einer Liste umfasst.

5. System zur Gewinnung von Informationen aus einem Radiologiebericht (74) auf der Grundlage von maschinellem Lernen, wobei das System umfasst:
eine Schnittstelle (70), die so konfiguriert ist, dass sie eine Frage zu einem Radiologiebericht (74) eines Patienten empfängt, wobei das Empfangen (10) das Empfangen (10) der Frage von dem Patienten umfasst oder wobei das Empfangen (10) das Empfangen (10) der Frage von einem behandelnden Arzt des Patienten umfasst und wobei die Schnittstelle (70) eine Benutzerschnittstelle ist;
eine Datenbank für medizinische Aufzeichnungen (72), in der der radiologische Bericht (74) des Patienten gespeichert ist;
einen Prozessor (76), der so konfiguriert ist, dass er:
Eingeben (12) der Frage und eines Radiologieberichts (74) aus der medizinischen Bildgebung des Patienten in ein System zur Verarbeitung natürlicher Sprache, wobei das System zur Verarbeitung natürlicher Sprache ein tiefes maschinelles Netzwerk umfasst, das so trainiert wurde, dass es zur Extraktion einer Antwort aus einer patientenspezifischen Eingabe beiträgt;
Bestimmen (14) einer Antwort auf die Frage als Reaktion auf das Eingeben (12) und unter Verwendung des tiefen maschinell erlernten Netzwerks, wobei die Antwort spezifisch für den Radiologiebericht (74) für den Patienten ist; und
wobei das tiefe maschinell erlernte Netzwerk mindestens einen ersten und einen zweiten tiefen maschinell erlernten Klassifikator umfasst, und wobei das Bestimmen (14) umfasst:
Extrahieren (15) des Inhalts, der Art der Frage, der Lage der Anatomie und der Maße aus der Frage durch den ersten tiefen maschinell erlernten Klassifikator und Analysieren der Frage, um den Inhalt, die Art der Frage, die Lage der Anatomie und die Maße zu verstehen,
Kopieren (16) des Radiologieberichts (74) in eine Schablone durch den zweiten tiefen maschinell erlernten Klassifikator, wobei die Schablone eine hierarchische standardisierte Struktur mit verschiedenen Ebenen umfasst, die mit einer Ebene mit Befunden endet, und wobei die Schablone aus dem Radiologiebericht auf der Grundlage der natürlichen Sprachverarbeitung ausgefüllt wird,
Auswählen (18) von Text aus der Schablone auf der Grundlage des Inhalts, der Art der Frage, der anatomischen Position und der Messungen, wobei der Inhalt, die Art der Frage, die anatomische Position und die Messungen in die Schablone eingepasst werden, um den entsprechenden Text als Beweis auszuwählen, und
Generieren (19) der Antwort aus dem ausgewählten Text, wobei die Frage in die Vorlage eingepasst wird, um die richtige Antwort auszuwählen, wobei der ausgewählte Text mit Erkenntnissen korreliert, die zur Beantwortung der Frage verwendet werden; und
eine Anzeige (80), die so konfiguriert ist, dass sie die Antwort, beispielsweise eine Antwort auf die Eingabe der Frage, auf der Benutzerschnittstelle ausgibt.

6. System nach Anspruch 5, wobei das tiefe maschinell erlernte Netzwerk (78) ein Netzwerk mit Langzeit-Kurzzeitgedächtnis umfasst.

## Revendications

1. Méthode mise en oeuvre par ordinateur pour obtenir des informations à partir d'un rapport radiologique (74) sur la base de l'apprentissage automatique, la méthode comprenant :
la réception (10) d'une question sur un patient à partir du matériel de l'interface (70), la réception (10) comprenant la réception (10) de la question du patient ou la réception (10) comprenant la réception (10) de la question d'un médecin traitant du patient et l'interface (70) étant une interface utilisateur ;
introduire (12) la question et un rapport radiologique (74) provenant de l'imagerie médicale du patient dans un système de traitement du langage naturel, le système de traitement du langage naturel comprenant un réseau d'apprentissage automatique profond ayant été formé pour contribuer à l'extraction d'une réponse à partir d'une entrée spécifique au patient ;
déterminer (14) une réponse à la question en réponse à la saisie (12) et à l'aide du réseau d'apprentissage automatique profond, la réponse étant spécifique au rapport radiologique (74) pour le patient ; et
l'édition (20) de la réponse, dans laquelle l'édition est destinée à un dispositif d'affichage, comme une réponse à la saisie de la question sur l'interface utilisateur,
dans lequel le réseau d'apprentissage automatique profond comprend au moins un premier et un deuxième classificateurs d'apprentissage automatique profond, et dans lequel la détermination (14) comprend :
extraction (15) du contenu, du type de question, de l'emplacement de l'anatomie et des mesures de la question par le premier classificateur d'apprentissage automatique profond et analyse de la question pour comprendre le contenu, le type de question, l'emplacement de l'anatomie et les mesures,
l'analyse syntaxique (16) du rapport radiologique (74) en un modèle par le classificateur de deuxième profondeur d'apprentissage automatique, dans lequel le modèle comprend une structure hiérarchique normalisée comprenant différents niveaux, se terminant par un niveau avec des résultats, et dans lequel le modèle est rempli à partir du rapport radiologique sur la base du traitement du langage naturel,
sélectionner (18) un texte dans le modèle en fonction du contenu, du type de question, de l'emplacement anatomique et des mesures, le contenu, le type de question, l'emplacement anatomique et les mesures étant mis en correspondance dans le modèle pour sélectionner le texte correspondant en tant que preuve, et
générer (19) la réponse à partir du texte sélectionné, la question étant mise en correspondance avec le modèle pour obtenir la bonne réponse, le texte sélectionné étant en corrélation avec les résultats utilisés pour répondre à la question.

2. La méthode selon la revendication 1, dans laquelle la détermination (14) comprend l'obtention (15) d'informations sur la question à partir de la question par le réseau d'apprentissage automatique profond
et/ou
dans lequel la détermination (14) comprend l'obtention (16) d'informations sur le rapport radiologique (74) à partir du rapport radiologique (74) par le réseau d'apprentissage automatique profond.

3. La méthode selon l'une des revendications précédentes, dans laquelle la détermination (14) comprend l'extraction (17) de phrases ou d'expressions du rapport de radiologie (74) par le réseau d'apprentissage automatique profond
et/ou
dans lequel la détermination (14) comprend l'extraction (18) d'éléments de preuve à partir de phrases ou d'expressions du rapport radiologique (74), les éléments de preuve étant extraits par le réseau d'apprentissage automatique profond .

4. La méthode selon l'une des revendications précédentes, dans laquelle la saisie (12) comprend la saisie (12) uniquement de la question et du rapport radiologique (74) et dans laquelle la détermination (14) comprend la détermination (14) de la réponse uniquement à partir des informations contenues dans le rapport radiologique (74),
ou
dans lequel la saisie (12) de la question comprend la saisie (12) de la question lorsque la question est différente de toute question utilisée pour former le réseau d'apprentissage automatique profond, et dans lequel la saisie (12) du rapport radiologique (74) comprend la saisie (12) du rapport radiologique (74) lorsque le rapport radiologique (74) est différent de tout rapport radiologique (74) utilisé pour former le réseau d'apprentissage automatique profond,
ou
dans lequel la saisie (12) de la question comprend la saisie (12) sous forme de texte sans sélection dans une liste.

5. Système permettant d'obtenir des informations à partir d'un rapport de radiologie (74) sur la base de l'apprentissage automatique, le système comprenant :
une interface (70) configurée pour recevoir une question sur un rapport radiologique (74) d'un patient, dans laquelle la réception (10) comprend la réception (10) de la question du patient ou dans laquelle la réception (10) comprend la réception (10) de la question d'un médecin traitant du patient et dans laquelle l'interface (70) est une interface utilisateur ;
une base de données de dossiers médicaux (72) contenant le rapport radiologique (74) du patient ;
un processeur (76) configuré pour :
introduire (12) la question et un rapport radiologique (74) provenant de l'imagerie médicale du patient dans un système de traitement du langage naturel, le système de traitement du langage naturel comprenant un réseau d'apprentissage automatique profond ayant été formé pour contribuer à l'extraction d'une réponse à partir d'une entrée spécifique au patient ;
déterminer (14) une réponse à la question en réponse à la saisie (12) et à l'aide du réseau d'apprentissage automatique profond, la réponse étant spécifique au rapport radiologique (74) pour le patient ; et
dans lequel le réseau d'apprentissage automatique profond comprend au moins un premier et un deuxième classificateurs d'apprentissage automatique profond, et dans lequel la détermination (14) comprend :
extraction (15) du contenu, du type de question, de l'emplacement de l'anatomie et des mesures de la question par le premier classificateur d'apprentissage automatique profond et analyse de la question pour comprendre le contenu, le type de question, l'emplacement de l'anatomie et les mesures,
l'analyse syntaxique (16) du rapport radiologique (74) en un modèle par le classificateur de deuxième profondeur d'apprentissage automatique, dans lequel le modèle comprend une structure hiérarchique normalisée comprenant différents niveaux, se terminant par un niveau avec des résultats, et dans lequel le modèle est rempli à partir du rapport radiologique sur la base du traitement du langage naturel,
sélectionner (18) un texte dans le modèle en fonction du contenu, du type de question, de l'emplacement anatomique et des mesures, le contenu, le type de question, l'emplacement anatomique et les mesures étant mis en correspondance dans le modèle pour sélectionner le texte correspondant en tant que preuve, et
générer (19) la réponse à partir du texte sélectionné, la question étant mise en correspondance avec le modèle pour obtenir la bonne réponse, le texte sélectionné étant en corrélation avec les résultats utilisés pour répondre à la question ; et
un écran (80) configuré pour afficher la réponse, par exemple une réponse à la question posée sur l'interface utilisateur.

6. Système selon la revendication 5, dans lequel le réseau d'apprentissage automatique profond (78) comprend un réseau à mémoire courte à long terme.
